# EUROPÄISCHE PATENTSCHRIFT

(11) **EP 3 601 145 B1**
(45) Veröffentlichungstag und Bekanntmachung des Hinweises auf die Patenterteilung: **10.09.2025**
(21) Anmeldenummer: 18721691.6
(22) Anmeldetag: 23.03.2018
(51) Int. Cl.: B67C 3/00

(54) **VORRICHTUNG ZUM BEFÜLLEN EINES BEHÄLTERS MIT EINEM FÜLLPRODUKT**
DEVICE FOR FILLING A CONTAINER WITH A FILLING PRODUCT
DISPOSITIF DE REMPLISSAGE D'UN CONTENANT AVEC UN PRODUIT DE REMPLISSAGE

(30) Priorität: 23.03.2017 DE 102017106337
(43) Veröffentlichungstag der Anmeldung: 05.02.2020
(60) Teilanmeldung: 20208177.4
(73) Patentinhaber: Krones AG, 93073 Neutraubling (DE)
(72) Erfinder: MUELLER, Holger, 93073 Neutraubling (DE); SOELLNER, Juergen, 93073 Neutraubling (DE)
(74) Vertreter: Nordmeyer, Philipp Werner
(86) Internationale Anmeldenummer: PCT/EP2018/057432
(87) Internationale Veröffentlichungsnummer: WO 2018/172519

(56) Entgegenhaltungen:
- EP-A1- 0 903 320
- EP-A2- 2 992 906
- DE-A1- 102014 109 447
- US-A- 5 771 917

## Beschreibung

### Technisches Gebiet

Die vorliegende Erfindung betrifft eine Vorrichtung zum Befüllen eines Behälters mit einem Füllprodukt, insbesondere eine Vorrichtung, in welcher ein Füllorgan zum Befüllen eines Behälters in einem Gehäuse aufgenommen ist.

### Technischer Hintergrund

Zum Befüllen von Behältern mit einem Füllprodukt ist eine Vielzahl unterschiedlicher Vorrichtungen bekannt, auf denen eine Vielzahl unterschiedlicher Füllverfahren ausgeführt wird. Den Füllvorrichtungen und Füllverfahren ist gemein, dass die jeweiligen füllproduktführenden Wege vor der Aufnahme der jeweiligen Produktion, turnusgemäß zu vorbestimmten Zeitpunkten, nach Wartungen und Reparaturen sowie bei einem Produktwechsel gereinigt werden müssen. Dies ist sowohl aus hygienischen Gründen notwendig, als auch um eine Produktreinheit des in den jeweiligen zu befüllenden Behälter zu füllenden Füllprodukts zu erreichen.

Zur Reinigung der Abfüllanlagen und insbesondere der produktführenden Wege ist es bekannt, eine so genannte "Cleaning in Place" - oder auch CIP-Reinigung - durchzuführen. Hierbei wird ohne vorherige Demontage der Füllproduktabfüllanlage eine vollständige Reinigung der füllproduktführenden Wege der Abfüllanlage sowie anderer produktberührter Bereiche, wie beispielsweise der bestimmte Anlagenbereiche einhausenden Gehäuse beziehungsweise Isolatoren, durchgeführt. Um eine effiziente Reinigung durchführen zu können, wird dabei das entsprechende Reinigungsmedium mehrfach wiederverwendet und mithin in einem Kreislauf geführt. Dazu ist in herkömmlichen Füllproduktabfüllanlagen ein CIP-Modul vorgesehen, in welchem das Reinigungsmedium angemischt und nach Verwendung wieder aufbereitet wird. Hierzu werden, je nach Reinigungsphase, in dem CIP-Modul beispielsweise Laugenkonzentrate, Säurekonzentrate, Tensidkonzentrate oder Desinfektionskonzentrate einem Prozesswasserstrom zugesetzt, und dann das auf diese Weise hergestellte Reinigungsmedium durch die entsprechenden füllproduktberührten und füllproduktführenden Bereiche der Füllproduktabfüllanlage geleitet.

Das auf diese Weise hergestellte Reinigungsmedium wird in herkömmlichen Füllproduktabfüllanlagen in einem Stapeltank zwischengespeichert, um einen hinreichend großen Puffer bereitzustellen, welcher das Zuführen des Reinigungsmediums in die zu reinigenden Anlagenbereiche ermöglicht und entsprechend ein schnelles und effizientes Abarbeiten eines Reinigungsprogramms ermöglicht.

Eine dem Stapeltank nachgeschaltete Pumpe sorgt dafür, dass zur Durchführung der Reinigung das Reinigungsmedium mit dem vorgesehen Druck und dem vorgesehenen Volumen durch die zu reinigenden Bereiche der Füllproduktabfüllanlage geführt wird, so dass eine zuverlässige und effiziente Reinigung erreicht wird. Das Reinigungsmedium fließt in dem Reinigungskreislauf sowohl durch Rohrleitungen, als auch in offenen Bereichen. In manchen Bereichen wird das Reinigungsmedium frei auf bestimmte Anlagenbereiche aufgebracht, beispielsweise über Spritzdüsen, um beispielsweise eine Innenreinigung eines einen Bereich der Füllproduktabfüllanlage einhausenden Gehäuses zu erreichen. Auch wird die Reinigung von Füllventilen häufig so durchgeführt, dass das Reinigungsmedium aus dem Füllventil austritt und dann in der Bodenwanne des jeweiligen, das Füllventil einhausenden Gehäuses aufgefangen wird. Entsprechend ist eine zweite Pumpe vorgesehen, mittels welcher das in der Bodenwanne des jeweiligen Gehäuses aufgefangene Reinigungsmedium wieder zurück zum CIP-Modul und dann nach Aufbereitung wieder in den Stapeltank gepumpt wird.

Die beiden Pumpen der herkömmlichen CIP-Vorrichtungen müssen gesteuert und überwacht werden, um zu verhindern, dass sich die beiden Pumpen gegeneinander aufschwingen und es so immer wieder zu Unterbrechungen des Reinigungsprozesses kommt, wenn beispielsweise der Puffertank leerläuft oder die Zufuhr des aus der Bodenwanne abgepumpten Reinigungsmediums zum CIP-Modul ausbleibt.

Mit anderen Worten müssen das Reinigungsmedienvolumen des Stapeltanks, welcher zur Zwischenpufferung des Reinigungsmediums dient, und das Reinigungsmedienvolumen in der Bodenwanne des Gehäuses miteinander im Gleichgewicht gehalten werden, da es sonst zum Aufschaukeln des Systems kommen kann.

Die EP 2 992 906 A2 beschreibt ein Verfahren zur Zwischensterilisation mindestens einer Oberfläche in einem Isolator einer Anlage zur Behandlung von Behältern. Die US 5,771,917 A beschreibt ein System zum Puffern und Abtransportieren aseptischer Produkte. Die DE10 2014 109 447 A1 beschreibt eine Vorrichtung und ein Verfahren zum Reinigen eines produktführenden Anlagenteils mittels eines Reinigungsmediums.

### Darstellung der Erfindung

Ausgehend von dem bekannten Stand der Technik ist es eine Aufgabe der vorliegenden Erfindung, eine Vorrichtung und ein Verfahren zum Befüllen eines Behälters mit einem Füllprodukt anzugeben, bei welcher ein weiter verbesserter Aufbau einer Reinigung vorgesehen ist.

Diese Aufgabe wird durch eine Vorrichtung mit den Merkmalen des Anspruchs 1 gelöst. Vorteilhafte Weiterbildungen ergeben sich aus der vorliegenden Beschreibung, den Figuren sowie den Unteransprüchen.

Entsprechend wird eine Vorrichtung zum Befüllen eines Behälters mit einem Füllprodukt vorgeschlagen, umfassend ein Gehäuse zur Aufnahme eines Füllorgans eines Füllers, wobei ein durch eine Bodenwanne des Gehäuses ausgebildeter Pufferbereich zum Puffern eines Reinigungsmediums für eine CIP-Reinigung vorgesehen ist, und einen CIP-Reinigungskreislauf, der zur Durchleitung des Reinigungsmediums durch füllproduktführende Bereiche des Füllers und/oder zur Zuleitung des Reinigungsmediums zu einer im Gehäuse angeordneten Reinigungsdüse ausgebildet ist. Erfindungsgemäß schließt der CIP-Reinigungskreislauf den Pufferbereich ein und ist ansonsten pufferfrei ausgebildet.

Unter einem Pufferbereich wird hierin ein Reservoir zur Aufnahme eines Teilvolumens des Reinigungsmediums verstanden, um eine temporäre Zwischenspeicherung dieses Teilvolumens des Reinigungsmediums bei seinem Durchfluss durch den CIP-Reinigungskreislauf zu erreichen.

Mit anderen Worten entkoppelt der Pufferbereich den Volumenstrom des Reinigungsmediums gegenüber dem übrigen CIP-Reinigungskreislauf. In den Pufferbereich hinein und aus dem Pufferbereich hinaus kann der identische Volumenstrom fließen. In bestimmten Betriebsphasen kann der Volumenstrom in den Pufferbereich hinein kleiner sein, als der Volumenstrom aus dem Pufferbereich heraus - so wie beispielsweise zu Beginn des Reinigungsbetriebs, bei dem durch das Füllen der zu reinigenden Fluidführungen mit dem Reinigungsmedium und durch das Benetzen der zu reinigenden Oberflächen zunächst nur ein geringerer Volumenstrom in den Pufferbereich hinein stattfindet. In weiteren Betriebsphasen kann der Volumenstrom in den Pufferbereich hinein größer sein, als aus dem Pufferbereich heraus - so wie beispielsweise bei einem gezielten Aufbau des Puffervolumens durch eine höhere Zufuhr an Reinigungsmedium in den Pufferbereich oder zum Ende des Reinigungsbetriebs hin, wenn das Reinigungsmedium noch aus den Fluidführungen nachfließt und Reinigungsmedium von den benetzten Oberflächen abfließt. Das im Pufferbereich aufgenommene Teilvolumen kann daher je nach Betriebsphase der Vorrichtung schwanken.

Das Reinigungsmedium steht, solange es im Pufferbereich aufgenommen ist, zur Reinigung der weiteren Anlagenkomponenten nicht zur Verfügung, sondern verweilt temporär im Pufferbereich. Bei dem im Pufferbereich zwischengespeicherten Teilvolumen des Reinigungsmediums handelt es sich aber nicht um einen vom übrigen CIP-Reinigungskreislauf separiertes (stehendes) Teilvolumen, sondern es findet während des Betriebs des CIP-Reinigungskreislaufs ein steter Strom an Reinigungsmedium durch den Pufferbereich statt, so dass das Teilvolumen des Pufferbereichs dynamisch umgewälzt wird.

Unter Pufferbereich werden insbesondere nicht die Rohrleitungen, Pumpen, Ventile und zu reinigenden Oberflächen der Vorrichtung und des CIP-Reinigungskreislaufs verstanden. Der Pufferbereich ist vielmehr ein dediziertes Reservoir zur Aufnahme eines Teilvolumens des Reinigungsmediums, das zur temporären Zwischenspeicherung des Reinigungsmediums dient.

Damit ergibt sich eine Führung des Reinigungsmediums im CIP-Reinigungskreislauf, die - ausgehend vom Pufferbereich - beispielsweise so beschrieben werden kann: Das Reinigungsmedium wird aus dem Pufferbereich in die füllproduktführenden Bereiche des Füllers sowie auf die füllproduktberührten Bereiche des Füllers und des Gehäuses gepumpt und fließt von diesen wieder in den Pufferbereich zurück. Hierzu sind eine Vielzahl von Rohrleitungen, Ventilen, Abzweigungen und anderen Fluidführungen vorgesehen, in denen eine Zwischenspeicherung des Reinigungsmediums aber nicht stattfindet. Eine Zwischenspeicherung des Reinigungsmediums findet vielmehr nur in dem einzigen Pufferbereich statt.

Der Pufferbereich kann ein den Betrieb des CIP-Reinigungskreislauf sicher ermöglichendes Teilvolumen umfassen, das bevorzugt im Bereich von 50% bis 300%, besonders bevorzugt von 100% bis 200%, des in den anderen Bereichen des CIP-Reinigungskreislaufs vorliegenden Reinigungsmedienvolumens ausmacht.

Der Pufferbereich kann auch so ausgestaltet sein, dass er das gesamte im CIP-Reinigungskreislauf zirkulierende Reinigungsmedium temporär aufnehmen kann. Dies kann zu Beginn oder zum Abschluss des Reinigungsbetriebs oder auch bei Unterbrechungen des Reinigungsbetriebs von Bedeutung sein, wenn die in den zu reinigenden fluidführenden Bereichen vorliegenden Reinigungsmedienvolumina in den Pufferbereich fließen und die auf den zu reinigenden Oberflächen haftenden Reinigungsmedienvolumina ebenfalls in den Pufferbereich abfließen. Der Pufferbereich ist entsprechend bevorzugt so dimensioniert, dass er das gesamte in dem CIP-Reinigungskreislauf vorliegende Reinigungsmedienvolumen aufnehmen kann, um ein versehentliches Austreten von Reinigungsmedium aus der Vorrichtung zu vermeiden.

Mit anderen Worten ist nur ein einziger Pufferbereich vorgesehen.

Dadurch, dass der CIP-Reinigungskreislauf den Pufferbereich einschließt und ansonsten pufferfrei ausgebildet ist, kann auf die Bereitstellung eines weiteren Puffers beziehungsweise Puffertanks verzichtet werden. Damit ergibt sich ein einfacher aufgebautes System für den CIP-Reinigungskreislauf, in welchem nur ein einziges zu überwachendes Volumen, nämlich der Pufferbereich, vorhanden ist. Damit vereinfacht sich die Reinigungssteuerung des CIP-Reinigungskreislaufs dahingehend, dass die gegenseitigen Abhängigkeiten zwischen den jeweils zu überwachenden Volumina, welche in herkömmlichen CIP-Reinigungskreisläufen vorhanden waren, nicht mehr auftreten und entsprechend die Anlagensteuerung vereinfacht wird. Mit anderen Worten kann durch eine einfache Überwachung des Pufferbereichs erreicht werden, dass der CIP-Reinigungskreislauf mit dem jeweiligen Reinigungsmedium zuverlässig betrieben wird und ein Leerlaufen von bestimmten Pufferbereichen auf diese Weise zuverlässiger vermieden werden kann.

Weiterhin kann vermieden werden, dass ein Aufschaukeln der Reinigungssteuerung der beiden, aus herkömmlichen CIP-Reinigungskreisläufen bekannten Volumina stattfindet. Damit kann die Reinigung durchgängig und effizient betrieben werden und Reinigungsunterbrechungen, wie sie aus den herkömmlichen CIP-Reinigungskreisläufen bekannt waren, können entsprechend vermieden werden.

Darüber hinaus kann durch die Verwendung eines einzelnen Pufferbereiches und einer ansonsten pufferfreien Ausbildung des CIP-Reinigungskreislaufs erreicht werden, dass das für die Reinigung einzusetzende Medienvolumen insgesamt reduziert werden kann. Damit sind die Kosten, welche für die jeweilige Reinigung der Vorrichtung zum Befüllen eines Behälters mit einem Füllprodukt anfallen, insgesamt reduziert und durch die Verringerung des bereitzustellenden Reinigungsmedienvolumens kann weiterhin die Reinigung schneller durchgeführt werden, da geringere Medienvolumen bereitgestellt und entsorgt werden müssen.

Der Anlagenaufbau kann ebenfalls vereinfacht ausgebildet werden und auf den separaten Puffertank kann verzichtet werden, was in einem kompakteren Anlagenaufbau resultiert, welcher auch einen kleineren Fußabdruck aufweist. Das Investitionsvolumen verringert sich ebenfalls.

Der Pufferbereich ist bevorzugt durch den Bodenbereich des jeweiligen Gehäuses bereitgestellt, welcher eine an sich übliche Ausbildung aufweisen kann. Mit anderen Worten sind in der Bodenwanne des Gehäuses keine weiteren Änderungen zur Ausbildung des Pufferbereichs notwendig und die Bodenwanne kann beispielsweise in Form einer herkömmlichen Gehäusewanne ausgebildet sein, welche auch in herkömmlichen Vorrichtungen zum Auffangen des Reinigungsmediums beziehungsweise zum Auffangen von übergelaufenen Füllproduktresten vorgesehen ist.

Die Bodenwanne des Gehäuses, welche als Pufferbereich ausgebildet ist, ist insbesondere plan mit Rand, wannenförmig, tischförmig oder in einer anderweitig herkömmlichen Ausgestaltung ausgebildet und bietet insbesondere keine weiteren Vertiefungen oder Absenkungen aus, welche für das Bereitstellen weiterer Aufnahmevolumina geeignet wären.

Bevorzugt ist eine einzige Umwälzpumpe im CIP-Reinigungskreislauf vorgesehen und der CIP-Reinigungskreislauf ist ansonsten pumpenfrei.

Mit anderen Worten ist eine einzige Umwälzpumpe in dem CIP-Reinigungskreislauf vorgesehen, mittels welcher die Umwälzung des Reinigungsmediums in dem gesamten CIP-Reinigungskreislauf betrieben werden kann. Die Umwälzpumpe entnimmt das Reinigungsmedium dabei aus dem Pufferbereich und führt das Reinigungsmedium dann durch die füllproduktführenden Wege und/oder in die Reinigungsdüsen zur Reinigung der füllproduktberührten Oberflächen.

Damit ergibt sich, dass lediglich eine einzige Pumpe angesteuert und betrieben werden muss, so dass Abhängigkeiten zwischen zwei Pumpen und ein mögliches Aufschwingen beziehungsweise unterschiedliche Regelzyklen zwischen zwei Pumpen, so wie sie in der herkömmlichen Reinigungsvorrichtung vorgesehen waren, vermieden werden können.

Damit kann die gesamte Vorrichtung insgesamt effizienter aufgebaut werden, da ein aufwändiges Regelverhalten beziehungsweise eine aufwändige Überwachung des Regelverhaltens zwischen zwei über zwei Puffervolumina miteinander verbundener Pumpen, so wie aus dem herkömmlichen Reinigungsvorrichtungen bekannt, vermieden werden kann. Der Anlagenaufbau kann ebenfalls vereinfacht ausgebildet werden, da auf eine weitere Pumpe verzichtet werden kann, so dass auch hier ein kompakterer Anlagenaufbau resultiert. Weiterhin resultiert die Ausgestaltung in einem geringeren Investitionsvolumen für den CIP-Reinigungskreislauf, da auf eine zweite Pumpe verzichtet werden kann und die Anlagensteuerung insgesamt vereinfacht wird.

Bevorzugt ist eine Medienzufuhr vorgesehen, mittels welcher Prozesswasser und/oder Reinigungskonzentrat und/oder Laugenkonzentrat und/oder Säurekonzentrat und/oder Desinfektionskonzentrat und/oder Tensidkonzentrat direkt in den CIP-Reinigungskreislauf eindosiert werden kann.

Mittels der Medienzufuhr kann entsprechend erreicht werden, dass die in der jeweiligen Reinigungsphase gewünschten oder geforderten Eigenschaften und Volumina des Reinigungsmediums in dem CIP-Reinigungskreislauf vorliegen.

Bevorzugt ist ein Temperatursensor zur Bestimmung der Temperatur des Reinigungsmediums in dem CIP-Reinigungskreislauf vorgesehen und ein Wärmetauscher ist zur Übergabe von Wärme an das in dem CIP-Reinigungskreislauf strömende Reinigungsmedium im CIP-Reinigungskreislauf vorgesehen, wobei eine Reinigungssteuerung den Wärmetauscher auf Grundlage der mittels des Temperatursensors bestimmten Temperatur des Reinigungsmediums ansteuert.

Durch das Vorsehen des Wärmetauschers ergibt sich die Möglichkeit, das Reinigungsmedium im CIP-Reinigungskreislauf auch auf die in der jeweiligen Reinigungsphase geforderte Temperatur zu bringen.

Ein einfacher Austausch des Reinigungsmediums und das Sicherstellen, dass immer ein frisches Reinigungsmedium verwendet wird, lässt sich durch einen Auslauf an dem CIP-Reinigungskreislauf erreichen, mittels dessen Reinigungsmedium aus dem CIP-Reinigungskreislauf auslaufen kann.

Besonders bevorzugt ist an dem Auslauf ein Wärmetauscher zur Übergabe eines Teils der Wärme des aus dem Auslauf auslaufenden Reinigungsmediums an in den CIP-Reinigungskreislauf zugeführtes Prozesswasser. So lässt sich die bereits im Reinigungsmedium vorliegende Wärme auch beim Verwerfen des bereits verwendeten Reinigungsmediums wiederverwenden und auf diese Weise eine effiziente Energieverwendung erreichen.

Bevorzugt weist der Puffer einen Füllhöhensensor auf, mittels dessen die Füllhöhe des im Puffer gepufferten Reinigungsmediums bestimmt werden kann, wobei der Füllhöhensensor mit einer Reinigungssteuerung kommuniziert, welche Medien auf Grundlage der bestimmten Füllhöhe über eine Medienzufuhr zum Erreichen oder Aufrechterhalten einer gewünschten Füllhöhe in den CIP-Reinigungskreislauf dosiert.

Auf diese Weise kann erreicht werden, dass das vorgegebene Puffervolumen im Puffer erreicht und aufrechterhalten wird.

Besonders bevorzugt ist ein Gütesensor zur Bestimmung der der Güte des Reinigungsmediums in dem CIP-Reinigungskreislauf vorgesehen und der Gütesensor kommuniziert mit einer Reinigungssteuerung, wobei die Reinigungssteuerung Prozesswasser und/oder Reinigungskonzentrat und/oder Laugenkonzentrat und/oder Säurekonzentrat und/oder Desinfektionskonzentrat und/oder Tensidkonzentrat auf Grundlage der mittels des Gütesensors bestimmten Güte des Reinigungsmediums Medien über eine Medienzufuhr in den CIP-Reinigungskreislauf dosiert.

Auf diese Weise lässt sich erreichen, dass das Reinigungsmedium die in der jeweiligen Reinigungsphase gewünschte oder geforderte Zusammensetzung aufweist oder erreicht.

Der Gütesensor dient zur Überwachung der Qualität des jeweils in dem Pufferbereich vorliegenden Reinigungsmediums. Der Gütesensor kann beispielsweise in Form eines pH-Sensors, eines Trübungssensors etc. vorgesehen sein.

Die oben genannte Aufgabe wird auch durch ein Verfahren mit den Merkmalen des Anspruchs 9 gelöst. Vorteilhafte Weiterbildungen ergeben sich aus der vorliegenden Beschreibung, den Figuren sowie den Unteransprüchen.

Entsprechend wird ein Verfahren zur Reinigung einer Vorrichtung zum Befüllen eines Behälters mit einem Füllprodukt vorgeschlagen. Die Vorrichtung umfasst ein Gehäuse zur Aufnahme eines Füllorgans eines Füllers, wobei ein durch eine Bodenwanne des Gehäuses ausgebildeter Pufferbereich zum Puffern eines Reinigungsmediums für eine CIP-Reinigung vorgesehen ist, und einen CIP-Reinigungskreislauf, der zur Durchleitung des Reinigungsmediums durch füllproduktführende Bereiche des Füllers und/oder zur Zuleitung des Reinigungsmediums zu einer im Gehäuse angeordneten Reinigungsdüse ausgebildet ist. Der CIP-Reinigungskreislauf schließt den Pufferbereich ein und ist ansonsten pufferfrei ausgebildet. Das Verfahren umfasst die Schritte des Beendens des Füllbetriebs, nach dem Beenden des Füllbetriebs des Befüllens des CIP-Reinigungskreislaufs mit Wasser zur Bereitstellung eines Reinigungsmediums im CIP-Reinigungskreislauf, gleichzeitig mit dem Befüllen oder nach Abschluss der Befüllung des CIP-Reinigungskreislaufs mit Wasser des Zudosierens von Reinigungsmedienkonzentrat zu dem im CIP-Reinigungskreislauf vorliegenden Reinigungsmedium, gleichzeitig mit dem Befüllen mit Wasser und/oder Zudosieren von Reinigungsmedienkonzentrat oder nach Abschluss der Befüllung und/oder Zudosierung des Erwärmens des im CIP-Reinigungskreislauf vorliegenden Reinigungsmediums auf eine vorgegebene Temperatur, des Zirkulierens des im CIP-Reinigungskreislauf vorliegenden Reinigungsmediums für eine vorgegebene Reinigungszeit, nach Ablauf der Reinigungszeit des Verwerfens des Reinigungsmediums.

Dadurch, dass der CIP-Reinigungskreislauf zunächst mit Wasser befüllt wird und dann die Temperatur und/die Güte des im CIP-Reinigungskreislauf vorliegenden Reinigungsmediums auf vorgegebene Werte gebracht wird, findet die Anmischung und Temperierung des Reinigungsmediums im CIP-Reinigungskreislauf statt. Entsprechend ist das Reinigungsmedium genau an die Vorgaben des CIP-Reinigungskreislauf angepasst, so dass hier eine besonders effiziente Herstellung des Reinigungsmediums erreicht werden kann. Mit anderen Worten ist eine Anmischung und Temperierung des Reinigungsmediums außerhalb des CIP-Reinigungskreislaufs nicht mehr notwendig. Damit entfallen alle Komponenten und Prozessschritte, die bei der Durchführung herkömmlicher Verfahren notwendig waren, so dass der Anlagenaufbau insgesamt kompakter und mit einem geringeren Investitionsvolumen ausgebildet sein kann.

Durch die Erwärmung des bereits im CIP-Reinigungskreislauf vorliegenden Reinigungsmediums findet zusammen mit der Erwärmung des Reinigungsmediums auch eine langsame Erwärmung der Anlagenkomponenten statt. Dies ist für die Anlagenkomponenten schonender, als das herkömmliche Einbringen eines bereits temperierten Reinigungsmediums.

Bevorzugt wird das verbrauchte Reinigungsmedium nach Ablauf der Reinigungszeit aus dem CIP-Reinigungskreislauf durch Zufuhr von Wasser zum CIP-Reinigungskreislauf verdrängt. Damit kann auf einfache Weise eine Verdrängung des Reinigungsmediums erreicht werden und die Anlage für den nächsten Reinigungsschritt vorbereitet werden.

Bevorzugt wird ein Teil der Wärmeenergie des aus dem CIP-Reinigungskreislauf verdrängten Reinigungsmediums an das zugeführte Wasser übergeben, bevorzugt mittels eines Wärmetauschers. Auf diese Weise kann ein Teil der einmal in das Reinigungsmedium eingebrachten Wärmeenergie weiter verwendet werden. Weiterhin kann durch die mit der teilweisen Übergabe der Wärmeenergie einhergehenden Erwärmung des zugeführten frischen Wassers vermieden werden, dass die Anlagenkomponenten durch die Beaufschlagung mit kaltem Wasser schlagartig abgekühlt werden. Damit kann das Reinigungsverfahren schonender ablaufen.

Die vorgegebene Temperatur und Güte des Reinigungsmediums wird für die vorgegebene Reinigungszeit bevorzugt aufrechterhalten und besonders bevorzugt wird eine Zufuhr von Wärmeenergie und/oder Reinigungsmedienkonzentrat zur Aufrechterhaltung der vorgegebenen Temperatur und/oder Güte durchgeführt. Damit kann über die gesamte Reinigungszeit hinweg die vorgegebene Reinigungswirkung aufrecht erhalten bleiben.

Bevorzugt umfasst der CIP-Reinigungskreislauf einen Pufferbereich, der beim Befüllen des CIP-Reinigungskreislaufs mit Wasser bis zu einem vorgegebenen Füllniveau befüllt wird. Damit ist eine Vorbefüllung des gesamten CIP-Reinigungskreislaufs möglich.

### Kurze Beschreibung der Figuren

Bevorzugte weitere Ausführungsformen der Erfindung werden durch die nachfolgende Beschreibung der Figuren näher erläutert. Dabei zeigen:
- Figur 1: eine schematische Darstellung einer Vorrichtung in einem ersten Ausführungsbeispiel und
- Figur 2: eine schematische Darstellung der Schaltung in einem zweiten exemplarischen Ausführungsbeispiel.

### Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente in den unterschiedlichen Figuren mit identischen Bezugszeichen versehen, und auf eine wiederholte Beschreibung dieser Elemente wird teilweise verzichtet, um Redundanzen zu vermeiden.

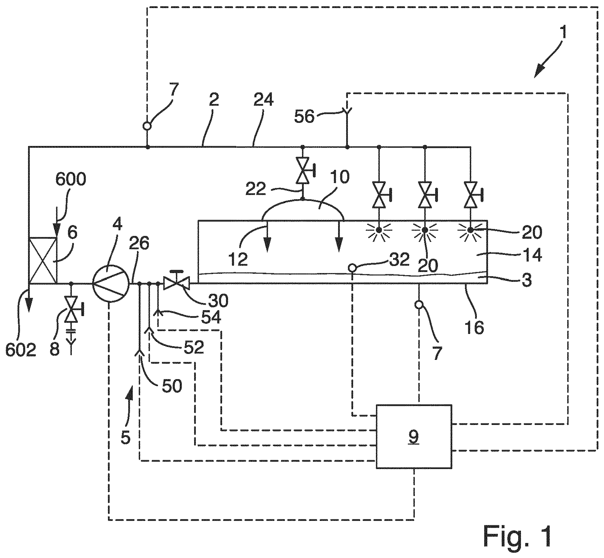

In Figur 1 ist schematisch eine Vorrichtung 1 zum Befüllen eines Behälters mit einem Füllprodukt gezeigt. Dazu ist ein Füller 10 in Form eines Rotationsfüllers vorgesehen, welcher schematisch angedeutete Füllorgane 12 aufweist, mittels welchen im Füllbetrieb ein Füllprodukt in die jeweils zu befüllenden Behälter eingeleitet wird. Zumindest die Füllorgane 12 des Füllers 10 sind in einem Gehäuse 14 aufgenommen.

Das Gehäuse 14 ist in dem in Figur 1 gezeigten Ausführungsbeispiel in Form eines hermetisch gegenüber der Umgebung abgeschlossenen Isolators ausgebildet, welcher ein aseptisches Füllen ermöglicht. Mit anderen Worten kann in dem als Isolator ausgebildeten Gehäuse 14 eine definierte Atmosphäre bereitgestellt werden, die insbesondere eine definierte Gaszusammensetzung und/oder eine definierte Temperatur aufweist und/oder eine definierte Keimkonzentration und/oder Partikelkonzentration nicht überschreitet. In einem Isolator für eine aseptische Abfüllung eines Füllprodukts herrscht weiterhin bevorzugt ein Überdruck gegenüber der Umgebung um das Eindringen von Umgebungsluft zu verhindern und entsprechend die definierte Atmosphäre in dem Gehäuse 14 aufrecht zu erhalten.

Das Gehäuse 14 kann aber auch in Form einer einfachen Einhausung des Füllers 10 vorgesehen sein, die in erster Linie einen (Eingreif-) Schutz für die Bediener bereitstellt. In einer solchen Ausbildung muss das Gehäuse 14 nicht vollständig geschlossen sein, sondern kann beispielsweise ohne Gehäusedach ausgebildet sein. Auch in einer solchen Ausbildung sind die Füllorgane 12 des Füllers 10 in Inneren des Gehäuses aufgenommen, um entsprechend einen Eingreifschutz in die sich bewegenden Teile des Füllers 10 für Bediener bereit zu stellen.

Das Gehäuse 14 weist eine Bodenwanne 16 auf, welche nach unten hin fluiddicht geschlossen ist, so dass Flüssigkeiten nach unten hin nicht austreten können. Eine solche Ausbildung einer Vorrichtung 1 zum Befüllen eines Behälters mit einem Füllprodukt, bei welcher die Füllorgane 12 innerhalb eines Gehäuses 14 angeordnet sind und der Boden des Gehäuses 14 in Form einer fluiddichten Bodenwanne 16 ausgebildet ist, ist prinzipiell bekannt - sowohl bei einer Ausbildung des Gehäuses 14 als Isolator als auch bei einer Ausbildung des Gehäuses 14 lediglich als Eingreifschutz.

Die Bodenwanne 16 kann dabei auf die üblicherweise verwendeten Formen zurückgreifen, also beispielsweise auf die Form einer im Wesentlichen kastenförmigen Bodenwanne, in Form eines Tischs, welcher beispielsweise mit dachförmigen Bereichen und daran angrenzenden Drainagerinnen ausgebildet ist, um ein Ablaufen von Füllproduktresten zu ermöglichen, welche durch ein Überlaufen oder Überschäumen von Füllprodukt aus den befüllten Behältern, oder beim Platzen oder anderen Behälterdefekten austretenden Füllproduktresten die Bodenwanne 16 erreichen. Über die Drainagerinnen können im Füllbetrieb die übergelaufenen Füllproduktreste gesammelt werden und entsprechend drainiert werden.

Um eine Reinigung der Vorrichtung 1 und insbesondere der füllproduktführenden Bereiche sowie der füllproduktberührten Bereiche der Vorrichtung 1 zu ermöglichen, welche turnusgemäß oder bei Füllproduktwechseln oder nach Wartungsarbeiten durchgeführt wird, ist ein CIP-Reinigungskreislauf 2 vorgesehen, mittels welchem ein Reinigungsmedium auf die jeweiligen zu reinigenden Flächen aufgebracht werden kann.

Der CIP-Reinigungskreislauf 2 kann beispielsweise über Reinigungsdüsen 20 verfügen, mittels welchen das Reinigungsmedium auf die im Gehäuse 14 vorliegenden und zu reinigenden Oberflächen - beispielsweise der Innenflächen des Gehäuses 14 und/oder der Außenflächen der Füllorgane 12 - aufgebracht werden kann.

Das Reinigungsmedium, das auf die zu reinigenden Oberflächen aufgebracht wird, fließt von diesen in die Bodenwanne 16 des Gehäuses 14 ab und wird in dieser gesammelt.

Der CIP-Reinigungskreislauf 2 kann beispielsweise über einen Innenreinigungszulauf 22 verfügen, mittels welchem das Reinigungsmedium in die füllproduktführenden Leitungen des Füllers 10 eingeleitet werden kann. Auf dieses Weise kann das Reinigungsmedium die füllproduktführenden Bereiche des Füllers 10 durchfließen und reinigen. Damit durchfließt das Reinigungsmedium auch die füllproduktführenden Bereiche der in das Gehäuse 14 hereinragenden Füllorgane 12. Auf diese Weise wird eine Innenreinigung des Füllers 10 durchgeführt.

Das Reinigungsmedium, das zur Innenreinigung des Füllers 10 verwendet wird, fließt aus den Füllventilen 12 in die Bodenwanne 16 des Gehäuses 14 - möglicherweise über dazwischen vorliegende Oberflächen oder Komponenten des Füllers 10 oder anderer Komponenten der Vorrichtung - und wird in dieser gesammelt.

Weiterhin ermöglicht der Innenreinigungszulauf 22 das Durchströmen der füllproduktführenden Bereiche, die dem eigentlichen Füller 10 vorgeschaltet sind, mit dem jeweiligen Reinigungsmedium. Der Innenreinigungszulauf 22 kann entsprechend auf ein Füllproduktreservoir oder andere dem Füller vorgeschaltete füllproduktführende Bereiche der Vorrichtung 1 durchfließen. Das Reinigungsmedium fließt dann schließlich ebenfalls aus den Füllventilen 12 in die Bodenwanne 16 des Gehäuses 14 - möglicherweise über dazwischen vorliegende Oberflächen des Füllers 10 oder anderer Komponenten der Vorrichtung - und wird in dieser gesammelt.

Der CIP-Reinigungskreislauf 2 sieht entsprechend vor, dass das Reinigungsmedium über einen Reinigungsmedienzulauf 24 dem Innenreinigungszulauf 22 und/oder den Reinigungsdüsen 20 zugeführt wird. Damit können sowohl die füllproduktführenden Leitungen und Bereiche des Füllers 10, als auch die jeweiligen innerhalb des Gehäuses 14 vorliegenden Oberflächen mit dem jeweiligen Reinigungsmedium beaufschlagt werden, um eine zuverlässige und vollständige Reinigung der füllproduktberührten und füllproduktführenden Bereiche zu erreichen, um die Vorrichtung 1 wieder in einen hygienisch einwandfreien Zustand zu versetzen und/oder einen Produktwechsel vorzubereiten.

Das in das Gehäuse 14 eintretende Reinigungsmedium wird im Bereich der Bodenwanne 16 aufgefangen, so dass hier ein Pufferbereich 3 ausgebildet wird. Mit anderen Worten dient die Bodenwanne 16 des Gehäuses 14 als Pufferbereich 3, in welchem das Reinigungsmedium aufgefangen wird und während der Reinigung zwischengepuffert werden kann.

Aus dem Pufferbereich 3 wird das Reinigungsmedium über einen Auslauf 30 des Pufferbereichs 3 abgezogen und mittels eines Reinigungsmedienvorlaufs 26 einer Umwälzpumpe 4 zugeführt. Die Umwälzpumpe 4 ist in dem CIP-Reinigungskreislauf 2 dazu vorgesehen, das Reinigungsmedium umzuwälzen. Entsprechend kann das im Pufferbereich 3 gepufferte Reinigungsmedium mittels der Umwälzpumpe 4 wieder in den Reinigungsmedienzulauf 24 gepumpt werden, um dann den Reinigungsdüsen 20 und/oder über den Innenreinigungszulauf 22 den füllproduktführenden Bereichen des Füllers 10 zugeführt zu werden.

Der CIP-Reinigungskreislauf 2 umfasst entsprechend sowohl den Pufferbereich 3 im Bereich der Bodenwanne 16 des Gehäuses 14, als auch die Umwälzpumpe 4, welche eine Umwälzung des Reinigungsmediums ermöglicht.

In dem gezeigten Ausführungsbeispiel stellt der Pufferbereich 3 ein Puffervolumen für das Reinigungsmedium von ca. 500l bereit, das im Verhältnis zu einem Reinigungsmedienvolumen von ca. 150l bis 200l im Bereich des übrigen CIP-Reinigungskreislaufs 2 steht. Das Reinigungsmedienvolumen des übrigen CIP-Reinigungskreislaufs setzt sich insbesondere durch die in dieser Ausführungsform gezeigten Volumina der Reinigungsdüsen 20, des Innenreinigungszulaufs 22, des Reinigungsmedienzulauf 24, des Reinigungsmedienvorlaufs 26 und der Umwälzpumpe 4 zusammen.

Stromabwärts des Pufferbereichs 3 münden Medienzuführungen 5 in den Reinigungsmedienvorlauf 26, wobei in der gezeigten Ausführungsform beispielsweise eine Prozesswasserzuführung 50, eine Laugenkonzentratzuführung 52 und eine Säurekonzentratzuführung 54 vorgesehen sind, mittels welcher sowohl eine Reinigungslauge als auch eine Reinigungssäure zusammen mit dem Prozesswasser angemischt und bereitgestellt werden kann. Eine weitere Medienzufuhr 5 in Form einer Tensidkonzentratzuführung 56 mündet in den Reinigungsmedienzulauf 24 und dient der Zufuhr von Tensiden.

Über die Medienzuführungen 5 können die für den jeweiligen Reinigungsschritt vorgesehenen Medien angemischt und bereitgestellt werden. Mittels der Nachdosierung entsprechender Medienkonzentrate kann das Reinigungsmedium im CIP-Reinigungskreislauf 2 aufgefrischt und/oder in seiner Zusammensetzung verändert werden, wenn dies für die Führung des Reinigungsverfahrens notwendig ist.

Damit ergibt sich, dass das Reinigungsmedium zur Reinigung sowohl der füllproduktführenden Bereiche des Füllers 10 als auch zur Reinigung der füllproduktberührten Bereiche des Füllers 10 und des Gehäuses 14 durch das Zuführen von Prozesswasser und dem gleichzeitigen Eindosieren von Laugenkonzentrat, Säurekonzentrat und/oder Tensiden bereitgestellt werden kann, wobei die Umwälzpumpe 4 dafür sorgt, dass die entsprechenden Volumina und Drücke, welche zur Reinigung vorgegeben sind, bereitgestellt werden.

Das Reinigungsmedium kann weiterhin über einen Wärmetauscher 6 auf die vorgegebene Reinigungsmedientemperatur erwärmt werden, so dass durch die Kreislaufführung des Reinigungsmediums im CIP-Reinigungskreislauf 2 mittels der Umwälzpumpe 4 das Reinigungsmedium nach und nach beim wiederholten Durchfließen des Wärmetauschers 6 auf die vorgegebene Temperatur gebracht werden kann.

Dadurch, dass das Reinigungsmedium mittels des Wärmetauschers 6 während seines Umlaufes und während des Umwälzens erwärmt wird, kann erreicht werden, dass sämtliche Anlagenkomponenten schonend erwärmt werden, was der Dauerhaltbarkeit der Anlage entgegenkommt. Bei herkömmlichen Anlagen wird ein bereits auf Temperatur gebrachtes Reinigungsmedium aus einem Stapeltank verwendet, so dass die Anlagenkomponenten mehr oder minder schlagartig erhitzt und durch den abrupten Temperaturanstieg belastet werden.

Dem Wärmetauscher 6 wird die an das Reinigungsmedium zu übergebende Wärme mittels einer Dampfzufuhr 600 zugeführt und das entstehende Kondensat wird über eine Kondensatableitung 602 wieder abgeleitet.

Mittels Temperatur- und/oder Gütesensoren 7, die beispielsweise in dem Reinigungsmedienzulauf 24 angeordnet sein können, kann sichergestellt werden, dass das Reinigungsmedium sowohl die geforderte Temperatur als auch die richtigen Konzentrationen an Lauge, Säure, Tensiden oder anderen Reinigungskomponenten aufweist. Sollte über die Temperatur- und/oder Gütesensoren 7 festgestellt werden, dass die jeweils geforderten Werte nicht erreicht werden, so wird über die entsprechenden Medienzuführungen 5 in das in dem CIP-Reinigungskreislauf 2 umgewälzte Reinigungsmedium nachdosiert, beispielsweise Prozesswasser und/oder Säure und/oder Lauge und/oder Tenside und/oder Desinfektionsmittel und/oder das in dem CIP-Reinigungskreislauf 2 umgewälzte Reinigungsmedium mittels des Wärmetauschers 6 nacherhitzt, um zu erreichen, dass das umgewälzte Reinigungsmedium den entsprechenden Vorgaben entspricht.

Die Nachdosierung und/oder Nacherhitzung wird über eine Reinigungssteuerung 9 gesteuert, welche ein Reinigungsprogramm durchfährt und welche die entsprechend vorgesehenen Werte des in dem CIP-Reinigungskreislauf 2 umgewälzten Reinigungsmediums mittels der Temperatur- und/oder Gütesensoren 7 überwacht.

Temperatur- und/oder Gütesensoren 7 können auch an anderen Positionen im CIP-Reinigungskreislauf 2 angeordnet sein, beispielsweise auch im Pufferbereich 3 des Gehäuses 14, wo ein Temperatur- und/oder Gütesensor 7 zusätzlich oder alternativ dargestellt ist.

Die Füllhöhe des Reinigungsmediums im Pufferbereich 3 im Gehäuse 14 der Vorrichtung 1 kann mittels eines Füllhöhensensors 32 gemessen werden. Mittels des Füllhöhensensors 32 kann entsprechend über die Reinigungssteuerung 9 der Zulauf von frischem Reinigungsmedium über die Medienzuführungen 5 gesteuert werden und dafür gesorgt werden, dass eine vorgegebene Füllhöhe nicht überschritten wird, um ein möglichst effizientes und Reinigungsmedien-sparendes Reinigen der Vorrichtung 1 zu erreichen.

Dadurch, dass der Pufferbereich 3, welcher im Bereich der Bodenwanne 16 des Gehäuses 14 ausgebildet ist, als der einzige Pufferbereich des CIP-Reinigungskreislaufes 2 ausgebildet ist und der Rest des CIP-Reinigungskreislaufes 2 pufferfrei ausgebildet ist, muss nur das Volumen des einzelnen Pufferbereichs 3 mit Reinigungsmedium gefüllt und dessen Füllhöhe überwacht werden.

Weiterhin wird durch die Verwendung nur einer einzigen Umwälzpumpe 4, wobei der restliche CIP-Reinigungskreislauf 2 pumpenfrei ausgebildet ist, erreicht, dass nur eine einzige Pumpe angesteuert zu werden braucht und entsprechend keine Interdependenzen zwischen dieser einen Pumpe und weiteren Pumpen auftreten. Entsprechend kann die Pumpensteuerung einfacher ausgebildet werden. Darüber hinaus kann durch die Verwendung einer einzigen Pumpe auch die Effizienz verbessert werden und die Komplexität des Anlagenaufbaus reduziert werden.

Nach Abschluss eines bestimmten Reinigungsabschnittes wird das verbrauchte Reinigungsmedium über einen Auslauf 8 verworfen. Mit anderen Worten wird das verbrauchte Reinigungsmedium mittels der Umwälzpumpe 4 aus dem Pufferbereich in den Auslauf 8 gepumpt und dort verworfen. Danach kann ein neues Reinigungsmedium angesetzt werden.

Unterschiedliche Reinigungsschritte können so nacheinander jeweils mit einem frischen Ansatz an Reinigungsmedium durchgeführt werden und der Prozess wird jeweils durch die Reinigungssteuerung 9 angesteuert.

So wird beispielsweise die Vorrichtung 1 zunächst mit Prozesswasser durchspült, um grobe Produktreste aus der Vorrichtung 1 heraus zu spülen, welche dann über den Auslauf 8 direkt ausgeleitet werden. In einem zweiten Prozessschritt wird beispielsweise ein Reinigungsmedium mit einem Zusatz von Laugekonzentrat oder Säurekonzentrat oder Tensidkonzentrat bereitgestellt, welches zur Reinigung der der produktführenden und der produktberührten Bereiche der Vorrichtung 1 verwendet wird.

In einem nachfolgenden Schritt kann die Vorrichtung 1 mit einem Desinfektionsmedium desinfiziert werden.

Entsprechend wird stets ein neuer Ansatz an Reinigungsmedium verwendet, so dass ein Eintrag von Fasern und Produktresten von einem Reinigungsschritt in einen späteren Reinigungsschritt vollständig vermieden wird und eine effiziente und hygienisch einwandfreie Reinigung, Desinfektion und Wiederherstellung der Vorrichtung 1 erreicht wird.

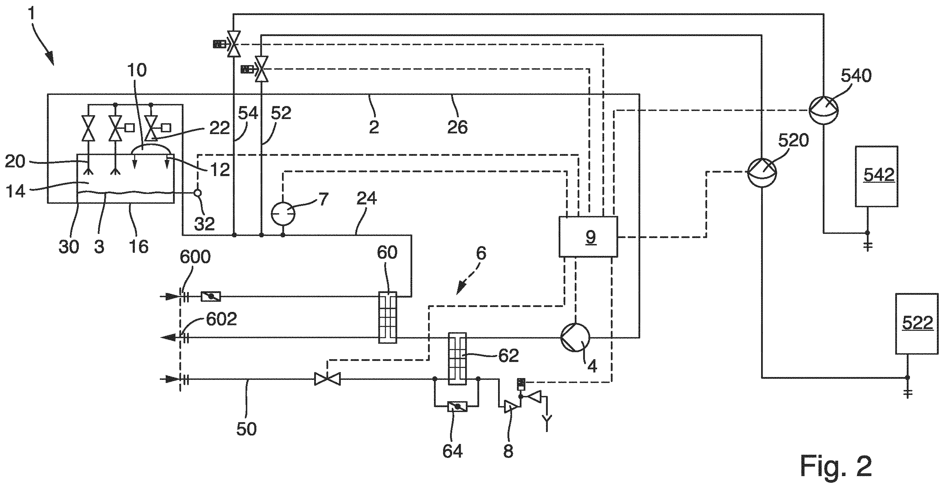

In Figur 2 ist ein Schaltbild einer weiteren Ausbildung der vorliegend beschriebenen Vorrichtung 1 gezeigt.

Die einzelnen in der Vorrichtung 1 verwendeten Komponenten entsprechen dabei im Wesentlichen den zur Figur 1 beschriebenen Komponenten.

In der Ausbildung der Figur 2 ist jedoch der Aufbau des Wärmetauschers 6 etwas differenzierter ausgestaltet. Insbesondere ist ein erster Wärmetauscher 60 vorgesehen, mittels welchem eine Erwärmung des Reinigungsmediums im CIP-Reinigungskreislauf 2 auf herkömmliche Weise erreicht werden kann. Die zur Erwärmung verwendete Wärmeenergie wird an diesen Wärmetauscher 60 über Prozessdampf geliefert. Der Prozessdampf wird mittels einer Dampfzufuhr 600 zugeführt und das entstehende Kondensat wird über eine Kondensatableitung 602 wieder abgeleitet.

Alternativ können auch andere Medien zur Zufuhr von Wärmeenergie zum Wärmetauscher 60 verwendet werden oder der Wärmetauscher 60 kann in Form einer elektrischen Heizung vorgesehen sein.

In dem Wärmetauscher 6 ist ein zweiter Wärmetauscher 62 vorgesehen, welcher mittels einer Bypassleitung 64 überbrückt werden kann. Der zweite Wärmetauscher 62 dient dazu, beim Verwerfen des Reinigungsmediums in den Auslauf 8 einen Teil der dem zu verwerfenden Reinigungsmedium vorhandenen Wärmeenergie an die Prozesswasserzufuhr 50 zu übergeben, um entsprechend die Wärmeenergie zum Vorwärmen des Prozesswassers zu verwenden, welches dann zum Ansatz eines nachfolgenden Reinigungsmediums verwendet wird.

Solange das Reinigungsmedium zur Reinigung mittels der Umwälzpumpe 4 umgewälzt wird, werden die beiden Seiten des zweiten Wärmetauschers 62 mittels der Bypassleitung 64 überbrückt, um den CIP-Reinigungskreislauf 2 zu schließen. Erst wenn das Reinigungsmedium auf den Auslauf 8 geleitet werden soll, wird die Bypassleitung 64 gemeinsam mit dem Auslauf 8 geöffnet, um ein Ausleiten des verbrauchten Reinigungsmediums aus dem CIP-Reinigungskreislauf 2 zu ermöglichen, wobei über den zweiten Wärmetauscher 62 entsprechend ein Teil der im verworfenen Reinigungsmedium vorhandenen Wärmeenergie an das neu anzusetzende Reinigungsmedium übertragen wird.

Entsprechend kann über diese Ausbildung ein besonders effizienter Wärmeaustausch erreicht werden und die Anlage insgesamt energieeffizient betrieben werden.

Die Laugenkonzentratzufuhr 52 ist ebenfalls etwas differenzierter dargestellt und es ist ein Laugenkonzentratbehälter 522 vorgesehen, in dem das Laugenkonzentrat vorgehalten wird. Das Laugenkonzentrat wird bei einer entsprechenden Anforderung durch die Reinigungssteuerung 9 mittels einer Laugenkonzentratdosierpumpe 520 in den Reinigungsmedienzulauf 54 des CIP-Reinigungskreislaufs 2 gepumpt. Die Laugenkonzentratdosierpumpe 520 ist nicht Teil des CIP-Reinigungskreislaufs 2, sondern eine separate Dosierpumpe. Die Laugenkonzentratdosierpumpe 520 könnte in einer weiteren Ausbildung auch in Form eines einfachen Zulaufs vorgesehen sein.

Auch die Säurekonzentratzufuhr 54 ist etwas differenzierter dargestellt und es ist ein Säurekonzentratbehälter 542 vorgesehen, in dem das Säurekonzentrat vorgehalten wird. Das Säurekonzentrat wird bei einer entsprechenden Anforderung durch die Reinigungssteuerung 9 mittels einer Säurekonzentratpumpe 540 in den Reinigungsmedienzulauf 54 des CIP-Reinigungskreislaufs 2 gepumpt. Die Säurekonzentratpumpe 540 ist nicht Teil des CIP-Reinigungskreislaufs 2, sondern eine separate Dosierpumpe. Die Säurekonzentratdosierpumpe 540 könnte in einer weiteren Ausbildung auch in Form eines einfachen Zulaufs vorgesehen sein.

Die Prozesswasserzufuhr 50 weist ein Prozesswasserventil 500 auf. Das Prozesswasserventil 500 dient dazu, die Zufuhr des Prozesswassers in den CIP-Kreislauf 2 zu regeln, um den CIP-Kreislauf 2 mit dem vorgesehenen Volumen an Prozesswasser zu befüllen. Weiterhin ist das Prozesswasserventil 500 nach dem Befüllen des CIP-Kreislaufs 2 mit Prozesswasser geschlossen, um im Reinigungsbetrieb sicher zu stellen, dass Reinigungsmedium nicht in die Prozesswasserleitung gedrückt wird.

Das mit den exemplarisch gezeigten Vorrichtungen 1 durchzuführende Reinigungsverfahren wird nachfolgend noch einmal exemplarisch beschrieben.

Insbesondere ist Verfahren zur Reinigung einer Vorrichtung zum Befüllen eines Behälters mit einem Füllprodukt, welche einen Füller 10 mit einem Füllorgan 12 und einen CIP-Reinigungskreislauf 2 zur Durchleitung eines Reinigungsmediums durch füllproduktführende Bereiche des Füllers 10 und/oder zur Zuleitung des Reinigungsmediums zu einer Reinigungsdüse 20 zur Außenreinigung des Füllers 10 vorgesehen.

Zunächst wird der Füllbetrieb beendet. Mit anderen Worten findet eine Reinigung nur dann statt, wenn nicht gleichzeitig Behälter mit Füllprodukt befüllt werden und die Vorrichtung 1 im Füllbetrieb ist.

Nachdem der Füllbetrieb beendet ist wird der CIP-Reinigungskreislauf 2 mit Wasser zur Bereitstellung eines Reinigungsmediums im CIP-Reinigungskreislauf 2 befüllt. Die Befüllung mit Wasser findet beispielsweise über die Prozesswasserzufuhr 50 statt.

Die Befüllung mit dem Prozesswasser wird beispielsweise beendet, wenn der Füllhöhensensor 32 eine vorgegebene Füllhöhe im Pufferbereich 3 detektiert. Alternativ kann auch das Volumen der Befüllung an der Prozesswasserzufuhr 50 überwacht werden und die Befüllung nach Erreichen eines vorbestimmten Volumens beendet werden.

Gleichzeitig mit dem Befüllen oder nach Abschluss der Befüllung des CIP-Reinigungskreislaufs 2 mit Wasser wird Reinigungsmedienkonzentrat zu dem im CIP-Reinigungskreislauf 2 vorliegenden Reinigungsmedium zudosiert - beispielsweise über die Laugenkonzentratzufuhr 52, die Säurekonzentratzufuhr 54 und/oder die Tensidkonzentratzufuhr 56. Die zuzuführenden Volumina an Reinigungsmediumkonzentrat werden beispielsweise über einen Gütesensor 7 ermittelt oder auf Grundlage von vorgegebenen Mischungsverhältnissen zudosiert.

Gleichzeitig mit dem Befüllen des CIP-Reinigungskreislaufs 2 mit Wasser und/oder dem Zudosieren von Reinigungsmedienkonzentrat oder aber nach Abschluss der Befüllung und/oder Zudosierung wird das im CIP-Reinigungskreislauf 2 vorliegende Reinigungsmedium auf eine vorgegebene Temperatur erwärmt. Die Erwärmung wird bevorzugt mittels des Wärmetauschers 6 beziehungsweise mittels der Kombination des ersten Wärmetauschers 60 und des zweiten Wärmetauschers 62 erreicht.

Das im CIP-Reinigungskreislauf 2 vorliegenden Reinigungsmediums wird dann für eine vorgegebene Reinigungszeit durch den CIP-Reinigungskreislauf 2 zirkuliert. Die Zirkulation wird durch die Umwälzpumpe 4 erreicht.

Nach Ablauf der Reinigungszeit wird das im CIP-Reinigungskreislauf 2 vorliegende und dann verbrauchte Reinigungsmedium verworfen. Dazu wird das Reinigungsmedium auf den Auslauf 8 geleitet.

Um das Reinigungsmedium effizient aus dem CIP-Reinigungskreislauf 2 herauszubringen, wird das verbrauchte Reinigungsmedium nach Ablauf der Reinigungszeit Zufuhr von frischen Prozesswasser über die Prozesswasserzufuhr 50 aus dem CIP-Reinigungskreislauf 2 verdrängt. Das dann im CIP-Reinigungskreislauf 2 vorliegende frische Wasser kann dann für einen weiteren Reinigungsschritt verwendet werden und entsprechend durch die Zufuhr von Reinigungsmedienkonzentraten und Wärme auf die dann gewünschten Eigenschaften des Reinigungsmediums gebracht werden.

Beim Verwerfen des Reinigungsmediums wird bevorzugt ein Teil der Wärmeenergie des aus dem CIP-Reinigungskreislauf verdrängten Reinigungsmediums an das zugeführte Prozesswasser übergeben, wobei hierzu der Wärmetauscher 62 vorgesehen ist.

Die vorgegebene Temperatur und Güte des Reinigungsmediums wird bevorzugt über die vorgegebene Reinigungszeit hinweg mittels des Temperatur- und Gütesensors 7 überwacht und entsprechend durch weitere Zufuhr von Wärmeenergie mittels des Wärmetauschers 6 und/oder der weiteren Zufuhr von Reinigungsmedienkonzentrat mittels der Medienzufuhr 5 aufrecht erhalten.

Die oben beschriebenen Prozessschritte des Reinigungsverfahrens und der Steuerung und Regelung der Vorrichtung 1 werden durch die Reinigungssteuerung 9 gesteuert. Die Reinigungssteuerung 9 kann entsprechend zur Ansteuerung der jeweiligen Komponenten der Vorrichtung 1 und zur Steuerung und Regelung der Prozessschritte programmiert sein, wobei die Reinigungssteuerung 9 sowohl vorgegebene Prozessschritte ansteuern kann, als auch die jeweiligen Anlagenkomponenten auf Grundlage von Sensorsignalen regeln kann.

### Bezugszeichenliste

- 1: Vorrichtung
- 10: Füller
- 12: Füllorgan
- 14: Gehäuse
- 16: Bodenwanne
- 2: CIP-Reinigungskreislauf
- 20: Reinigungsdüse
- 22: Innenreinigungszulauf
- 24: Reinigungsmedienzulauf
- 26: Reinigungsmedienvorlauf
- 3: Pufferbereich
- 30: Auslauf
- 32: Füllhöhensensor
- 4: Umwälzpumpe
- 5: Medienzufuhr
- 50: Prozesswasserzufuhr
- 52: Laugenkonzentratzufuhr
- 54: Säurekonzentratzufuhr
- 56: Tensidkonzentratzufuhr
- 500: Prozesswasserventil
- 520: Laugenkonzentratdosierpumpe
- 522: Laugenkonzentratbehälter
- 540: Säurekonzentratdosierpumpe
- 542: Säurekonzentratbehälter
- 6: Wärmetauscher
- 60: erster Wärmetauscher
- 62: zweiter Wärmetauscher
- 64: Bypassleitung
- 600: Dampfzufuhr
- 602: Kondensatableitung
- 7: Temperatur- und Gütesensor
- 8: Auslauf
- 9: Reinigungssteuerung

## Patentansprüche

1. Vorrichtung (1) zum Befüllen eines Behälters mit einem Füllprodukt, umfassend ein Gehäuse (14) zur Aufnahme eines Füllorgans (12) eines Füllers (10), wobei ein durch eine Bodenwanne (16) des Gehäuses (14) ausgebildeter Pufferbereich (3) zum Puffern eines Reinigungsmediums für eine CIP-Reinigung vorgesehen ist, und einen CIP-Reinigungskreislauf (2), der zur Durchleitung des Reinigungsmediums durch füllproduktführende Bereiche des Füllers (10) und/oder zur Zuleitung des Reinigungsmediums zu einer im Gehäuse (14) angeordneten Reinigungsdüse (20) ausgebildet ist, wobei
der CIP-Reinigungskreislauf (2) den Pufferbereich (3) einschließt und ansonsten pufferfrei ausgebildet ist.

2. Vorrichtung (1) gemäß Anspruch 1, **dadurch gekennzeichnet, dass** eine einzige Umwälzpumpe (4) im CIP-Reinigungskreislauf (2) vorgesehen ist und der CIP-Reinigungskreislauf (2) ansonsten pumpenfrei ist.

3. Vorrichtung (1) gemäß Anspruch 1 oder 2, **dadurch gekennzeichnet, dass** eine Medienzufuhr (5) vorgesehen ist, mittels welcher Prozesswasser und/oder Reinigungskonzentrat und/oder Laugenkonzentrat und/oder Säurekonzentrat und/oder Desinfektionskonzentrat und/oder Tensidkonzentrat direkt in den CIP-Reinigungskreislauf (2) eindosiert werden kann.

4. Vorrichtung (1) gemäß einem der vorstehenden Ansprüche, **dadurch gekennzeichnet, dass** ein Temperatursensor (7) zur Bestimmung der Temperatur des Reinigungsmediums in dem CIP-Reinigungskreislauf (2) vorgesehen ist und ein Wärmetauscher (6) zur Übergabe von Wärme an das in dem CIP-Reinigungskreislauf (2) strömende Reinigungsmedium im CIP-Reinigungskreislauf (2) vorgesehen ist, wobei eine Reinigungssteuerung (9) den Wärmetauscher (6) auf Grundlage der mittels des Temperatursensors (7) bestimmten Temperatur des Reinigungsmediums ansteuert.

5. Vorrichtung (1) gemäß einem der vorstehenden Ansprüche, **dadurch gekennzeichnet, dass** ein Auslauf (8) an dem CIP-Reinigungskreislauf (2) vorgesehen ist, um Reinigungsmedium aus dem CIP-Reinigungskreislauf (2) auslaufen zu lassen.

6. Vorrichtung (1) gemäß Anspruch 5, **dadurch gekennzeichnet, dass** an dem Auslauf (8) ein Wärmetauscher (62) zur Übergabe eines Teils der Wärme des aus dem Auslauf auslaufenden Reinigungsmediums an in den CIP-Reinigungskreislauf (2) zugeführtes Prozesswasser.

7. Vorrichtung (1) gemäß einem der vorstehenden Ansprüche, **dadurch gekennzeichnet, dass** der Puffer (3) einen Füllhöhensensor (32) aufweist, mittels dessen die Füllhöhe des im Puffer (3) gepufferten Reinigungsmediums bestimmt werden kann, wobei der Füllhöhensensor (32) mit einer Reinigungssteuerung (9) kommuniziert, welche Medien auf Grundlage der bestimmten Füllhöhe über eine Medienzufuhr (5) zum Erreichen oder Aufrechterhalten einer gewünschten Füllhöhe in den CIP-Reinigungskreislauf (2) dosiert.

8. Vorrichtung (1) gemäß einem der vorstehenden Ansprüche, **dadurch gekennzeichnet, dass** ein Gütesensor (7) zur Bestimmung der der Güte des Reinigungsmediums in dem CIP-Reinigungskreislauf (2) vorgesehen ist und der Gütesensor mit einer Reinigungssteuerung (9) kommuniziert, wobei die Reinigungssteuerung (9) Prozesswasser und/oder Reinigungskonzentrat und/oder Laugenkonzentrat und/oder Säurekonzentrat und/oder Desinfektionskonzentrat und/oder Tensidkonzentrat auf Grundlage der mittels des Gütesensors (7) bestimmten Güte des Reinigungsmediums Medien über eine Medienzufuhr (5) in den CIP-Reinigungskreislauf (2) dosiert.

9. Verfahren zur Reinigung einer Vorrichtung (1) zum Befüllen eines Behälters mit einem Füllprodukt, welche ein Gehäuse (14) zur Aufnahme eines Füllorgans (12) eines Füllers (10), wobei ein durch eine Bodenwanne (16) des Gehäuses (14) ausgebildeter Pufferbereich (3) zum Puffern eines Reinigungsmediums für eine CIP-Reinigung vorgesehen ist, und einen CIP-Reinigungskreislauf (2) zur Durchleitung des Reinigungsmediums durch füllproduktführende Bereiche des Füllers (10) und/oder zur Zuleitung des Reinigungsmediums zu einer im Gehäuse (14) angeordneten Reinigungsdüse (20) zur Außenreinigung des Füllers (10) umfasst, wobei der CIP-Reinigungskreislauf (2) den Pufferbereich (3) einschließt und ansonsten pufferfrei ausgebildet ist, und
das Verfahren die Schritte umfasst:
o Beenden des Füllbetriebs
∘ nach Beenden des Füllbetriebs: Befüllen des CIP-Reinigungskreislaufs (2) mit Wasser zur Bereitstellung eines Reinigungsmediums im CIP-Reinigungskreislauf (2)
∘ gleichzeitig mit dem Befüllen oder nach Abschluss der Befüllung des CIP-Reinigungskreislaufs (2) mit Wasser:
∘ Zudosieren von Reinigungsmedienkonzentrat zu dem im CIP-Reinigungskreislauf (2) vorliegenden Reinigungsmedium (2)
∘ gleichzeitig mit dem Befüllen mit Wasser und/oder Zudosieren von Reinigungsmedienkonzentrat oder nach Abschluss der Befüllung und/oder Zudosierung: Erwärmen des im CIP-Reinigungskreislauf (2) vorliegenden Reinigungsmediums auf eine vorgegebene Temperatur
∘ Zirkulieren des im CIP-Reinigungskreislauf (2) vorliegenden Reinigungsmediums für eine vorgegebene Reinigungszeit
∘ Nach Ablauf der Reinigungszeit: Verwerfen des Reinigungsmediums.

10. Verfahren gemäß Anspruch 9, **dadurch gekennzeichnet, dass** das verbrauchte Reinigungsmedium nach Ablauf der Reinigungszeit aus dem CIP-Reinigungskreislauf (2) durch Zufuhr von Wasser zum CIP-Reinigungskreislauf (2) verdrängt wird.

11. Verfahren gemäß Anspruch 10, **dadurch gekennzeichnet, dass** ein Teil der Wärmeenergie des aus dem CIP-Reinigungskreislauf verdrängten Reinigungsmediums an das zugeführte Wasser übergeben wird, bevorzugt mittels eines Wärmetauschers (62).

12. Verfahren gemäß einem der Ansprüche 9 bis 11, **dadurch gekennzeichnet, dass** die vorgegebene Temperatur und Güte des Reinigungsmediums für die vorgegebene Reinigungszeit aufrechterhalten wird und bevorzugt eine Zufuhr von Wärmeenergie und/oder Reinigungsmedienkonzentrat zur Aufrechterhaltung der vorgegebenen Temperatur und/oder Güte durchgeführt wird.

13. Verfahren gemäß einem der Ansprüche 9 bis 12, **dadurch gekennzeichnet, dass** der CIP-Reinigungskreislauf (2) einen Pufferbereich (3) umfasst, der beim Befüllen des CIP-Reinigungskreislaufs (2) mit Wasser bis zu einem vorgegebenen Füllniveau befüllt wird.

## Claims

1. An apparatus (1) for filling a container with a filling product, comprising a housing (14) for receiving a filling element (12) of a filler (10), wherein a buffer region (3) formed by a base pan (16) of the housing (14) is provided for buffering a cleaning medium for CIP cleaning, and a CIP cleaning circuit (2) which is configured to pass the cleaning medium through filling product-carrying regions of the filler (10) and/or to supply the cleaning medium to a cleaning nozzle (20) arranged in the housing (14), wherein
the CIP cleaning circuit (2) includes the buffer region (3) and is otherwise buffer-free.

2. The apparatus (1) according to claim 1, **characterized in that** a single circulation pump (4) is provided in the CIP cleaning circuit (2) and the CIP cleaning circuit (2) is otherwise pump-free.

3. The apparatus (1) according to claim 1 or 2, **characterized in that** a media supply (5) is provided with which process water and/or cleaning concentrate and/or alkaline concentrate and/or acid concentrate and/or disinfectant concentrate and/or surfactant concentrate can be dosed directly into the CIP cleaning circuit (2).

4. The apparatus (1) according to any of the preceding claims,
**characterized in that** a temperature sensor (7) is provided for determining the temperature of the cleaning medium in the CIP cleaning circuit (2) and a heat exchanger (6) is provided in the CIP cleaning circuit (2) for delivering heat to the cleaning medium flowing in the CIP cleaning circuit (2), wherein a cleaning control system (9) controls the heat exchanger (6) on the basis of the temperature of the cleaning medium determined with the temperature sensor (7).

5. The apparatus (1) according to any of the preceding claims,
**characterized in that** an outlet (8) is provided on the CIP cleaning circuit (2) to allow cleaning medium to flow out of the CIP cleaning circuit (2).

6. The apparatus (1) according to claim 5, **characterized in that** a heat exchanger (62) is at the outlet (8) for delivering part of the heat of the cleaning medium flowing out of the outlet to process water fed into the CIP cleaning circuit (2).

7. The apparatus (1) according to any of the preceding claims,
**characterized in that** the buffer (3) has a fill level sensor (32) with which the fill level of the cleaning medium buffered in the buffer (3) can be determined, wherein the fill level sensor (32) communicates with a cleaning control system (9) which doses media into the CIP cleaning circuit (2) on the basis of the determined fill level via a media supply (5) in order to achieve or maintain a desired fill level.

8. The apparatus (1) according to any of the preceding claims,
**characterized in that** a quality sensor (7) is provided for determining the quality of the cleaning medium in the CIP cleaning circuit (2) and the quality sensor communicates with a cleaning control system (9), wherein the cleaning control system (9) doses process water and/or cleaning concentrate and/or alkaline concentrate and/or acid concentrate and/or disinfectant concentrate and/or surfactant concentrate into the CIP cleaning circuit (2) via a media supply (5) on the basis of the quality of the cleaning medium determined by the quality sensor (7).

9. A method for cleaning an apparatus (1) for filling a container with a filling product, which apparatus comprises a housing (14) for receiving a filling element (12) of a filler (10), wherein a buffer region (3) formed by a base pan (16) of the housing (14) is provided for buffering a cleaning medium for CIP cleaning, and a CIP cleaning circuit (2) which is configured to pass the cleaning medium through filling product-carrying regions of the filler (10) and/or to supply the cleaning medium to a cleaning nozzle (20) arranged in the housing (14) for external cleaning of the filler (10), wherein the CIP cleaning circuit (2) includes the buffer region (3) and is otherwise configured to be buffer-free, and
the method comprises the steps of:
∘ ending the filling operation
∘ once the filling operation is completed: filling the CIP cleaning circuit (2) with water to provide a cleaning medium in the CIP cleaning circuit (2)
∘ simultaneously with the filling of the CIP cleaning circuit (2) with water or after completion of the filling:
∘ adding cleaning medium concentrate to the cleaning medium (2) present in the CIP cleaning circuit (2)
∘ simultaneously with the filling of water and/or adding cleaning medium concentrate or after completion of the filling and/or adding process: heating the cleaning medium present in the CIP cleaning circuit (2) to a predetermined temperature
o circulating the cleaning medium present in the CIP cleaning circuit (2) for a predetermined cleaning time
∘ after the cleaning time has elapsed: discarding the cleaning medium.

10. The method according to claim 9, **characterized in that** the used cleaning medium is displaced from the CIP cleaning circuit (2) after the cleaning time has elapsed by supplying water to the CIP cleaning circuit (2).

11. The method according to claim 10, **characterized in that** part of the thermal energy of the cleaning medium displaced from the CIP cleaning circuit is delivered to the supplied water, preferably by a heat exchanger (62).

12. The method according to any of claims 9 to 11, **characterized in that** the predetermined temperature and quality of the cleaning medium is maintained for the predetermined cleaning time and preferably a supply of thermal energy and/or cleaning medium concentrate is carried out to maintain the predetermined temperature and/or quality.

13. The method according to any of claims 9 to 12, **characterized in that** the CIP cleaning circuit (2) comprises a buffer region (3) which is filled up to a predetermined fill level when the CIP cleaning circuit (2) is filled with water.

## Revendications

1. Dispositif (1) permettant de remplir un récipient avec un produit de remplissage, comprenant un boîtier (14) pour la réception d'un organe de remplissage (12) d'une remplisseuse (10), dans lequel une zone tampon (3) réalisée par une cuve de fond (16) du boîtier (14) est prévue pour tamponner un milieu de nettoyage pour un nettoyage CIP, et un circuit de nettoyage CIP (2), qui est réalisé pour le passage du milieu de nettoyage à travers des zones de la remplisseuse (10) guidant le produit de remplissage et/ou pour l'amenée du milieu de nettoyage à une buse de nettoyage (20) disposée dans le boîtier (14), dans lequel
le circuit de nettoyage CIP (2) inclut la zone tampon (3) et est par ailleurs réalisé sans tampon.

2. Dispositif (1) selon la revendication 1, **caractérisé en ce qu'**une seule pompe de circulation (4) est prévue dans le circuit de nettoyage CIP (2) et le circuit de nettoyage CIP (2) est par ailleurs dépourvu de pompe.

3. Dispositif (1) selon la revendication 1 ou 2, **caractérisé en ce qu'**il est prévu une amenée de milieu (5) à l'aide de laquelle de l'eau de processus et/ou du concentré de nettoyage et/ou du concentré de lessive et/ou du concentré d'acide et/ou du concentré de désinfection et/ou du concentré de tensioactif peuvent être dosés directement dans le circuit de nettoyage CIP (2).

4. Dispositif (1) selon l'une des revendications précédentes,
**caractérisé en ce qu'**un capteur de température (7) est prévu pour la détermination de la température du milieu de nettoyage dans le circuit de nettoyage CIP (2) et un échangeur de chaleur (6) est prévu dans le circuit de nettoyage CIP (2) pour le transfert de chaleur au milieu de nettoyage s'écoulant dans le circuit de nettoyage CIP (2), dans lequel une commande de nettoyage (9) commande l'échangeur de chaleur (6) sur la base de la température du milieu de nettoyage déterminée à l'aide du capteur de température (7).

5. Dispositif (1) selon l'une des revendications précédentes,
**caractérisé en ce qu'**une sortie (8) est prévue sur le circuit de nettoyage CIP (2) pour permettre au milieu de nettoyage de s'écouler hors du circuit de nettoyage CIP (2).

6. Dispositif (1) selon la revendication 5, **caractérisé en ce qu'**un échangeur de chaleur (62) est prévu à la sortie (8) pour le transfert d'une partie de la chaleur du milieu de nettoyage sortant de la sortie à de l'eau de traitement alimentée dans le circuit de nettoyage CIP (2).

7. Dispositif (1) selon l'une des revendications précédentes,
**caractérisé en ce que** le tampon (3) présente un capteur de niveau (32) à l'aide duquel le niveau du milieu de nettoyage tamponné dans le tampon (3) peut être déterminé, dans lequel le capteur de niveau (32) communique avec une commande de nettoyage (9) qui dose des milieux sur la base du niveau déterminé dans le circuit de nettoyage CIP (2) par le biais d'une alimentation en milieu (5) pour l'atteinte ou le maintien d'un niveau souhaité.

8. Dispositif (1) selon l'une des revendications précédentes,
**caractérisé en ce qu'**un capteur de qualité (7) est prévu pour la détermination de la qualité du milieu de nettoyage dans le circuit de nettoyage CIP (2) et le capteur de qualité communique avec une commande de nettoyage (9), dans lequel la commande de nettoyage (9) dose de l'eau de processus et/ou du concentré de nettoyage et/ou du concentré de lessive et/ou du concentré d'acide et/ou du concentré de désinfection et/ou du concentré de tensioactif sur la base de la qualité du milieu de nettoyage déterminée à l'aide du capteur de qualité (7), par l'intermédiaire d'une alimentation en milieu (5) dans le circuit de nettoyage CIP (2).

9. Procédé pour le nettoyage d'un dispositif (1) permettant de remplir un récipient avec un produit de remplissage, qui comprend un boîtier (14) pour la réception d'un organe de remplissage (12) d'une remplisseuse (10), dans lequel une zone tampon (3) réalisée par une cuve de fond (16) du boîtier (14) est prévue pour tamponner un milieu de nettoyage pour un nettoyage CIP, et comprend un circuit de nettoyage CIP (2) pour le passage du milieu de nettoyage à travers des zones de la remplisseuse (10) guidant le produit de remplissage et/ou pour l'amenée du milieu de nettoyage à une buse de nettoyage (20) disposée dans le boîtier (14) pour le nettoyage extérieur de la remplisseuse (10), dans lequel le circuit de nettoyage CIP (2) inclut la zone tampon (3) et est par ailleurs réalisé sans tampon, et
le procédé comprend les étapes :
∘ d'arrêt du mode de remplissage
∘ après l'arrêt du mode de remplissage : de remplissage du circuit de nettoyage CIP (2) avec de l'eau pour la fourniture d'un milieu de nettoyage dans le circuit de nettoyage CIP (2)
∘ en même temps que le remplissage ou à la fin du remplissage du circuit de nettoyage CIP (2) avec de l'eau :
∘ d'ajout de concentré de milieu de nettoyage au milieu de nettoyage (2) présent dans le circuit de nettoyage CIP (2)
∘ en même temps que le remplissage avec de l'eau et/ou l'ajout de concentré de milieu de nettoyage ou après la fin du remplissage et/ou de l'ajout : de chauffage du milieu de nettoyage présent dans le circuit de nettoyage CIP (2) à une température prédéfinie
∘ de circulation du produit de nettoyage présent dans le circuit de nettoyage CIP (2) pendant une durée de nettoyage prédéfinie
∘ après l'écoulement de la durée de nettoyage : de rejet du milieu de nettoyage.

10. Procédé selon la revendication 9, **caractérisé en ce que** le milieu de nettoyage usé est déplacé hors du circuit de nettoyage CIP (2) après l'écoulement de la durée de nettoyage, par alimentation en eau au circuit de nettoyage CIP (2).

11. Procédé selon la revendication 10, **caractérisé en ce qu'**une partie de l'énergie thermique du milieu de nettoyage déplacé hors du circuit de nettoyage CIP est transférée à l'eau alimentée, de préférence à l'aide d'un échangeur de chaleur (62).

12. Procédé selon l'une des revendications 9 à 11, **caractérisé en ce que** la température et la qualité prédéfinies du milieu de nettoyage sont maintenues pendant la durée de nettoyage prédéfinie et, de préférence, une alimentation en énergie thermique et/ou en concentré de milieu de nettoyage est effectuée pour le maintien de la température et/ou la qualité prédéfinies.

13. Procédé selon l'une des revendications 9 à 12, **caractérisé en ce que** le circuit de nettoyage CIP (2) comprend une zone tampon (3) qui est remplie d'eau jusqu'à un niveau de remplissage prédéfini lors du remplissage du circuit de nettoyage CIP (2).
